# EUROPÄISCHE PATENTSCHRIFT

(11) **EP 2 327 441 B1**
(45) Veröffentlichungstag und Bekanntmachung des Hinweises auf die Patenterteilung: **23.01.2013**
(21) Anmeldenummer: 09176992.7
(22) Anmeldetag: 25.11.2009
(51) Int. Cl.: A61M 16/06, A61M 16/01

(54) **System zur Beatmung von Patienten**
System for ventilating patients
Système de ventilation de patients

(43) Veröffentlichungstag der Anmeldung: 01.06.2011
(73) Patentinhaber: Dräger Medical GmbH, 23558 Lübeck (DE)
(72) Erfinder: Koulechov, Kirill, Dr., 23669, Lübeck (DE); Tappehorn, Ludger, 23560, Lübeck (DE); Wallnewitz, Oliver, 23556, Lübeck (DE)
(74) Vertreter: UEXKÜLL & STOLBERG

(56) Entgegenhaltungen:
- EP-A1- 2 078 534
- WO-A1-2005/009521
- DE-U1- 20 217 885
- US-A1- 2003 075 182
- US-A1- 2008 178 875
- US-A1- 2009 223 521

## Beschreibung

Die vorliegende Erfindung betrifft ein System zur Beatmung von Patienten.

Beatmungsvorrichtungen werden zur nicht-invasiven Beatmung von Patienten eingesetzt. Die Beatmungsvorrichtungen umfassen eine Beatmungsmaske, die auf den Gesichtsbereich des Patienten aufgelegt wird und im Allgemeinen sowohl die Nase als auch den Mund abdeckt, so dass der Patient bei einem Atmen durch die Nase und den Mund mit künstlicher Atemluft über eine längere Zeitspanne versorgt werden kann. Beatmungsvorrichtungen mit einer Beatmungsmaske umfassen außerdem im Allgemeinen eine Stirnstütze und ein Bandsystem.

Außerdem können Beatmungsvorrichtungen auch Anästhesiemasken aufweisen. Anästhesiemasken werden ebenfalls auf den Gesichtsbereich des Patienten aufgelegt, so dass die Nase als auch den Mund abdeckt abgedeckt ist. Beatmungsvorrichtungen mit Anästhesiemasken werden nur kurzzeitig auf den Gesichtsbereich des Patienten aufgelegt und umfassen deshalb kein Bandsystem zur dauerhaften Befestigung an dem Kopf des Patienten wie Beatmungsmasken sowie auch keine Stirnstütze. In nachteiliger Weise sind deshalb für die künstliche Beatmung von Patienten Beatmungsvorrichtungen mit Beatmungsmasken und für die Anästhesie Beatmungsvorrichtungen mit Anästhesiemasken vorzuhalten. Dies erfordert somit einen hohen Aufwand zur Vorhaltung und Herstellung von den beiden unterschiedlichen Beatmungsvorrichtungen.

Aus der US 2003/0034034 A1 ist eine Beatmungsvorrichtung bekannt. Die Beatmungsvorrichtung mit einer Beatmungsmaske umfasst eine Stirnstütze, so dass die Beatmungsvorrichtung lediglich als Beatmungsmaske, nicht jedoch als Anästhesiemaske nutzbar ist. Dokument US2009/0223521 A1 offenbart ein Beatmungssystem mit einer Maske, die an einen Luftschlauch angeschlossen werden kann, mit einer Stirnstütze, die eine Auflagefläche zum Auflegen der Stirnstütze auf die Stirn eines Patienten aufweist, und mit einem Bandsystem einschließlich Befestigungsmitteln zum Befestigen der Maske an dem Kopf eines Patienten. Die Stirnstütze und die Befestigungsmittel sind dabei lösbar mit der Maske verbunden und die Position der Auflagefläche der Stirnstütze ist gegenüber der Maske einstellbar.

Dokument W02005/009521 A1 offenbart eine Stirnstütze für Beatmungsmasken mit einer Maske, die eine Anschlusseinrichtung für einen Luftschlauch aufweist, mit einer Stirnstütze zur Anlage an die Stirn eines Patienten und mit einem Bandsystem einschließlich Befestigungsmitteln zur Fixierung der Maske an dem Kopf eines Patienten. Außerdem offenbart das Dokument, dass die Stirnstütze ein aufblasbares elastisches Element aufweist, das zur Anlage an die Stirn eines Benutzers vorgesehen ist.

Die Aufgabe der vorliegenden Erfindung besteht deshalb darin, ein System zur Beatmung von Patienten Verfügung zu stellen, das mit einem geringen Aufwand sowohl als Beatmungsmaske als auch als Anästhesiemaske eingesetzt werden kann. Das System zur Beatmung von Patienten soll ferner in der Handhabung einfach und zuverlässig sein und geringe Herstellungskosten aufweisen.

Diese Aufgabe wird gelöst mit einem System zur Beatmung von Patienten oder einer Beatmungsvorrichtung, umfassend eine Maske, eine an der Maske ausgebildete Anschlusseinrichtung zur Verbindung der Maske mit einem Luftschlauch, eine Stirnstütze mit einer Auflagefläche zum Auflegen auf eine Stirn eines Patienten, ein Bandsystem zur Befestigung der Maske an einem Kopf eines Patienten, Befestigungsmittel für das Bandsystem, wobei die Stirnstütze und die Befestigungsmittel lösbar an der Maske befestigbar sind, so dass das System ohne Stirnstütze und ohne Befestigungsmittel, nur mit der Maske, als Anästhesiemaske nutzbar ist und das System mit Stirnstütze und mit Befestigungsmittel als Beatmungsmaske nutzbar ist.

Die Position der Auflagefläche der Stirnstütze relativ zu der Maske, ist fixierbar, einstellbar.

Die Stirnstütze weist eine im Volumen veränderliche, mit Luft gefüllte Auflageeinrichtung auf, so dass mittels eines veränderbaren Volumens an Luft in der Auflageeinrichtung die Position der Auflagefläche der Stirnstütze relativ zu der Maske einstellbar ist.

Die Auflageeinrichtung ist ein elastischer Ballon und die Auflageeinrichtung umfasst ein Ventil zum Befüllen und Entleeren des Ballons.

Die Komponenten des Systems, nämlich die Maske, die Anschlusseinrichtung, die Stirnstütze, das Bandsystem und die Befestigungsmittel, können somit miteinander kombiniert werden, so dass bei der Verwendung sämtlicher Komponenten eine Beatmungsmaske vorliegt und bei der Verwendung nur eines Teils der Komponenten eine Anästhesiemaske vorliegt. Die nicht verwendeten Komponenten werden an einem andern Ort aufbewahrt für eine spätere Verwendung von sämtlichen Komponenten.

Der Ballon ist aus einem elastischen Material gefertigt und liegt somit auf der Stirn des Patienten auf. Damit kann in vorteilhafter Weise die von der Stirnstütze, das heißt dem Ballon, auf die Stirn aufgebrachte Kraft besonders gleichmäßig auf die Stirnfläche des Patienten übertragen werden. Aufgrund des Ventils kann der Ballon dabei auch mit Luft gefüllt und entleert werden, so dass dadurch das Volumen veränderbar ist und die Stirnstütze, das heißt die Auflagefläche der Stirnstütze relativ zu der Maske einstellbar ist und somit die Stirnstütze an unterschiedliche Gesichtsformen vom Patienten angepasst werden kann.

Die Stirnstütze und die Befestigungsmittel, insbesondere sämtliche Befestigungsmittel, können von der Maske gelöst werden. Damit kann die Maske bei einer Verwendung mit der Stirnstütze und den Befestigungsmitteln als Beatmungsmaske eingesetzt werden. Eine Beatmungsmaske erfordert eine Stirnstütze und Befestigungsmittel, weil an den Befestigungsmitteln ein Bandsystem befestigbar ist zur dauerhaften Fixierung der Beatmungsmaske am Gesichtsbereich eines künstlich zu beatmenden Patienten. Beatmungsmasken werden aufgrund der künstlichen Beatmung über einen längeren Zeitraum am Gesichtsbereich des Patienten befestigt, so dass eine Stirnstütze erforderlich ist, damit im Nasenbereich keine Verletzungen aufgrund der Beatmungsmaske auftreten. Anästhesiemasken werden nur für einen kurzen Zeitraum auf den Gesichtsbereich des Patienten aufgelegt, um über eine kurze Zeitspanne den Patienten mit Anästhesiegas zu versorgen. Aus diesem Grund benötigen Anästhesiemasken keine Stirnstütze und auch keine Befestigungsmittel sowie auch kein Bandsystem, das an dem Befestigungsmittel fixiert bzw. befestigt ist. Anästhesiemasken werden dabei im Allgemeinen nur vom Anästhesiearzt von Hand auf den Gesichtsbereich für einen kurzen Zeitraum aufgelegt und auch von Hand am Gesichtsbereich für die Versorgung mit Anästhesiegas gehalten.

In einer weiteren Ausführungsform sind die Stirnstütze und die Befestigungsmittel, insbesondere sämtliche Befestigungsmittel, als ein separates Bauteil ausgebildet und/oder sämtliche Befestigungsmittel sind lösbar an der Maske befestigbar.

In einer zusätzlichen Ausgestaltung ist das Bauteil an der Maske lösbar befestigbar. Die Stirnstütze und sämtliche Befestigungsmittel sind an dem separaten Bauteil ausgebildet. Bei einer Verwendung des Systems zur Beatmung von Patienten als Beatmungsmaske ist das Bauteil an der Maske befestigt, so dass dadurch das System zur Beatmung von Patienten auch die Stirnstütze und die Befestigungsmittel umfasst. Somit können an den Befestigungsmitteln auch das Bandsystem befestigt werden und es liegt damit ein System mit einer Beatmungsmaske vor. Zur Verwendung des Systems als Anästhesiemaske, das heißt bei einer Verwendung der Maske als Anästhesiemaske, braucht lediglich das Bauteil aufgrund der lösbaren Verbindung mit der Maske von der Maske entfernt werden, so dass lediglich die Maske als Anästhesiemaske ohne der Stirnstütze und den Befestigungsmitteln vorliegt. Diese Maske als Anästhesiemaske kann dann von einem Anästhesiearzt auf den Gesichtsbereich des Patienten aufgelegt werden. Damit ist es in vorteilhafter Weise möglich, dass mit dem System sowohl eine Anästhesiemaske als auch eine Beatmungsmaske zur Verfügung gestellt werden kann, aufgrund der lösbaren Befestigung der Stirnstütze und der Befestigungsmittel an der Maske.

In einer Variante umfasst das Bauteil einen Ring. Der Ring stellt somit eine Fixierungseinrichtung zur Fixierung des Bauteils an der Maske dar. Die Fixierungseinrichtung kann dabei außer als Ring auch als eine Schraub- oder Rastverbindung oder Klemmverbindung ausgebildet sein.

In einer zusätzlichen Ausgestaltung umfasst die Maske einen Stutzen, insbesondere als Anschlusseinrichtung zur Verbindung der Maske mit dem Luftschlauch. Der Stutzen stellt somit eine Gegenfixierungseinrichtung dar, und mittels der Gegenfixierungseinrichtung an der Maske kann die Fixierungseinrichtung an dem Bauteil befestigt werden. Die an der Maske ausgebildete Gegenfixierungseinrichtung kann dabei auch beispielsweise ein Gewinde oder eine Ausnehmung für eine Rastverbindung sein.

In einer ergänzenden Ausführungsform ist der Ring des Bauteils auf den Stutzen aufschiebbar zur lösbaren Verbindung des Bauteils mit der Maske.

In einer weiteren Variante sind die Stirnstütze und die Befestigungsmittel mittels einer Schraub- oder Rastverbindung an der Maske lösbar befestigbar. Die Schraub- oder Rastverbindung stellt somit eine Fixierungsvorrichtung dar.

Vorzugsweise sind die Befestigungsmittel als wenigstens ein Haken und/oder wenigstens eine Öse und/oder wenigstens ein Schlitz ausgebildet.

In einer zusätzlichen Ausführungsform umfasst das Bandsystem ein Stirnband und Seitenbänder.

In einer ergänzenden Ausgestaltung ist das Bandsystem elastisch.

In einer weiteren Ausführungsform sind die Maske und/oder die Stirnstütze und/oder das Bandsystem wenigstens teilweise, insbesondere vollständig, aus Kunststoff ausgebildet.

Zweckmäßig sind die Befestigungsmittel ausschließlich an dem separaten Bauteil ausgebildet.

Vorzugsweise besteht das Bandsystem wenigstens teilweise aus einem Gewebe, insbesondere aus Kunstfasern.

Im Nachfolgenden wird ein Ausführungsbeispiel der Erfindung unter Bezugnahme auf die beigefügte Zeichnung näher beschrieben. Es zeigt:
- Fig. 1: eine Seitenansicht eines System zur Beatmung von Patienten,
- Fig. 2: eine Vorderansicht einer Stirnstütze und Befestigungsmittel als separates Bauteil des Systems gemäß Fig. 1 und
- Fig. 3: eine Seitenansicht des Bauteiles gemäß Fig. 2.

Systeme 1 zur Beatmung von Patienten werden zur nicht-invasiven künstlichen Beatmung von Patienten und für die Anästhesie von Patienten eingesetzt. Das System 1 zur Beatmung von Patienten umfasst eine Maske 2 mit einer Anschlusseinrichtung 5 als Stutzen 6 zum Anschluss und lösbaren Fixierung eines nicht dargestellten Luftschlauches. Die Maske 2 kann aufgrund ihrer Geometrie sowohl die Nase als auch den Mund eines Patienten abdecken, das heißt gleichzeitig die Nase und den Mund künstlich beatmen.

Die Maske 2 kann sowohl als Beatmungsmaske 3 zur künstlichen Beatmung von Patienten als auch als Anästhesiemaske 4 für die Anästhesie eingesetzt werden. Anästhesiemasken 4 werden nur kurzzeitig auf den Gesichtsbereich eines Patienten aufgelegt und während des Auflegens von der Hand eines Anästhesiearztes gehalten. Bei einer Verwendung der Maske 2 des Systems 1 zur Beatmung von Patienten als Anästhesiemaske 4, umfasst das System 1 zur Beatmung von Patienten somit keine Stirnstütze 7 als auch kein Bandsystem 10. Bei einer Verwendung der Maske 2 des Systems 1 als Beatmungsmaske 3 umfasst das System 1 die Stirnstütze 7 und das Bandsystem 10.

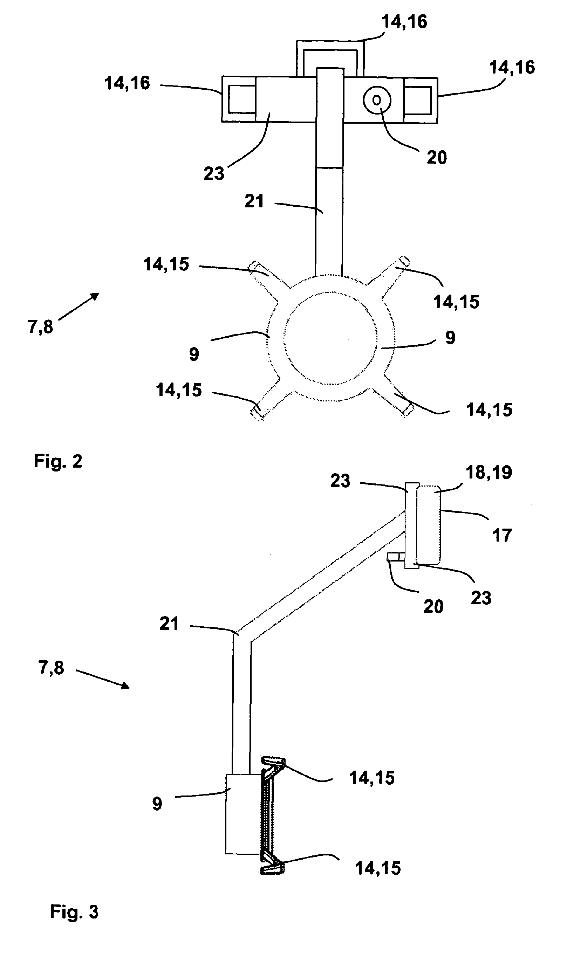

Die im Wesentlichen aus Kunststoff ausgebildete Stirnstütze 7 umfasst einen Stirnstützenarm 21. An einem unteren Ende des Stirnstützenarmes 21 ist ein Ring 9 mit Haken 15 als Befestigungsmitteln 14 angeordnet (Fig. 2). Am oberen Ende des Stützenarmes 21 ist eine Stützenplatte 23 angeordnet (Fig. 2 und 3). An der Stützenplatte 23 sind Ösen 16 als Befestigungsmittel 14 ausgebildet. Außerdem ist an der Stützenplatte 23 eine als Ballon 19 ausgebildete Auflageeinrichtung 18 vorhanden. Die Auflageeinrichtung 18, das heißt der Ballon 19 mit einer Auflagefläche 17, dient dazu, auf einer Stirn eines zu beatmenden Patienten aufgelegt zu werden, um auf die Stirn mittels der Auflagefläche 17 Kräfte übertragen zu können. Die Stirnstütze 7 ist ferner an der Stützenplatte 23 mit einem Ventil 20 versehen. Mittels des Ventils 20 kann Luft in den elastischen Ballon 19 aus Kunststoff eingefüllt und entnommen werden, so dass dadurch das Volumen des Ballons 19 veränderbar ist. Auf diese Weise kann die relative Ausrichtung der Auflagefläche 17 der Stirnstütze 7 bezüglich der Maske 2 verändert werden und damit die Stirnstütze 7 an unterschiedliche Formen des Kopfes eines Patienten angepasst werden. Die Ausbildung der Auflageeinrichtung 18 als Ballon 19 ermöglicht ferner in vorteilhafter Weise eine sehr gleichmäßige Übertragung der Kräfte an der Auflagefläche 17 auf die Stirn des Patienten, so dass dadurch in vorteilhafter Weise an der Stirn des Patienten keine Verletzungen aufgrund von lokal hohen Kräften auftreten können. Der Ring 9 der Stirnstütze 7 dient als Fixierungseinrichtung zur Fixierung der Stirnstütze 7 an der Maske 2, sofern die Maske 2 als Beatmungsmaske 3 eingesetzt wird. Hierzu wird in einfacher Weise lediglich der Ring 9 der Stirnstütze 7 auf den Stutzen 6 als Anschlusseinrichtung 5 für den Luftschlauch der Maske 2 aufgeschoben, bevor der Luftschlauch an dem Stutzen 6 befestigt wird. Zum Abnehmen der Stirnstütze 7 von der Maske 2 braucht lediglich der Ring 9 von dem Stutzen 6 abgeschoben werden.

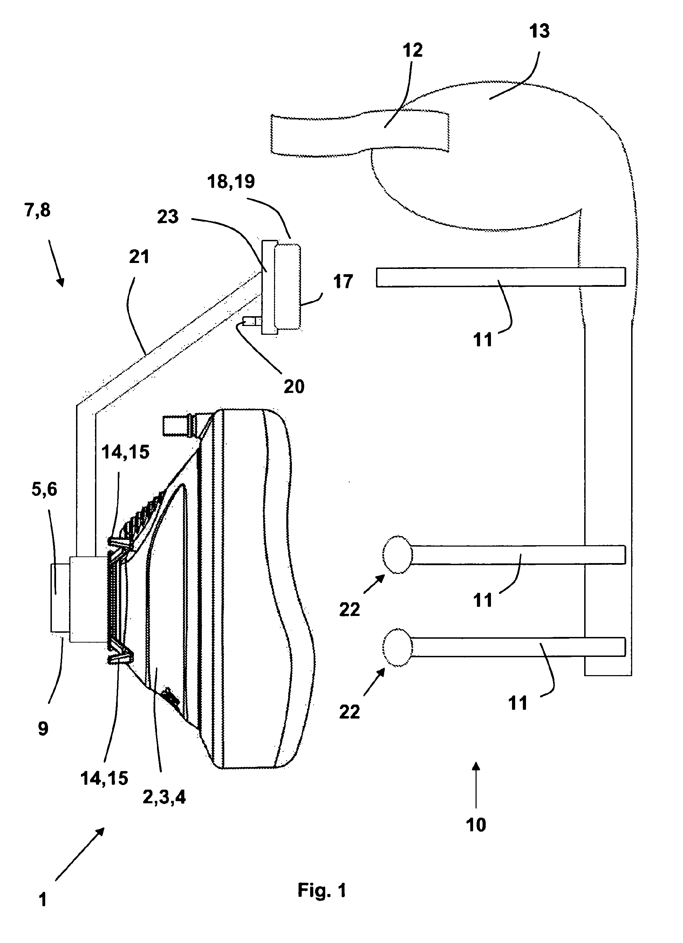

Bei der Verwendung der Maske 2 des System 1 zur Beatmung von Patienten als Beatmungsmaske 3, umfasst das System 1 auch ein Bandsystem 10 (Fig. 1). Das Bandsystem 10 umfasst dabei sechs Seitenbänder 11, von denen aufgrund der Perspektive in Fig. 1 nur drei sichtbar sind sowie ein Stirnband 12. Das Stirnband 12 ist dabei an einem großflächigen Flächenelement 13 des Bandsystems 10 befestigt. Zur dauerhaften Befestigung der Beatmungsmaske 3 an einer Gesichtsfläche eines künstlich zu beatmenden Patienten werden Befestigungslaschen 22 der Seitenbänder 10 an den Haken 15 des Ringes 9 eingehängt. Das Stirnband 12 und die beiden oberen Seitenbänder 11 weisen dabei einen nicht dargestellten Klettverschluss auf, mittels dem nach dem Einführen des Stirnbandes 12 und der Seitenbänder 11 in die Ösen 16 an der Stützenplatte 23 an dem übrigen Seitenband 11 bzw. dem übrigen Stirnband 12 befestigt werden können, so dass dadurch die beiden oberen Seitenbänder 11 und das Stirnband 12 an den Ösen 16 der Stützenplatte 23 befestigt sind. Damit kann die Beatmungsmaske 3 dauerhaft am Gesichtsbereich eines Patienten befestigt werden. Das Bandsystem 10 ist außerdem elastisch, so dass sich dadurch das Bandsystem 10 an unterschiedliche Größen und Kopfformen von Patienten anpassen kann.

Insgesamt betrachtet, sind mit dem erfindungsgemäßen System 1 zur Beatmung von Patienten wesentliche Vorteile verbunden. Die Maske 2 des Systems 1 kann sowohl als Beatmungsmaske 3 als auch als Anästhesiemaske 4 genutzt werden. Damit brauchen für eine Beatmungsmaske 2 als auch eine Anästhesiemaske 4 als Bestandteil des Systems 1 zur Beatmung von Patienten nicht unterschiedliche Masken 2 vorgehalten werden, sondern mit einer Maske 2 kann diese entweder sowohl als Anästhesiemaske 4 ohne der Stirnstütze 7 und ohne dem Bandsystem 10 eingesetzt werden und bei einer Verwendung als Beatmungsmaske 3 mit der Stirnstütze 7 und mit dem Bandsystem 10. Dabei kann die Stirnstütze 7 in einfacher Weise mit dem Ring 9 auf den Stutzen 6 aufgeschoben werden, so dass in sehr kurzer Zeit die Maske 2 von einer Anästhesiemaske 4 in eine Beatmungsmaske 3 umgewandelt werden kann. Hohe Kosten für das Herstellen unterschiedlicher Masken 2 als Beatmungsmaske 3 und Anästhesiemaske 4 können damit vermieden werden und andererseits braucht beispielsweise in Krankenhäusern lediglich eine Maske 2 vorgehalten werden, die sowohl als Beatmungsmaske 3 als auch als Anästhesiemaske 4 genutzt werden kann.

### BEZUGSZEICHENLISTE

- 1: System zur Beatmung von Patienten
- 2: Maske
- 3: Beatmungsmaske
- 4: Anästhesiemaske
- 5: Anschlusseinrichtung
- 6: Stutzen
- 7: Stirnstütze
- 8: Bauteil
- 9: Ring
- 10: Bandsystem
- 11: Seitenband
- 12: Stirnband
- 13: Flächenelement
- 14: Befestigungsmittel
- 15: Haken
- 16: Öse
- 17: Auflagefläche
- 18: Auflageeinrichtung
- 19: Ballon
- 20: Ventil
- 21: Stirnstützenarm
- 22: Befestigungslasche
- 23: Stützenplatte

## Patentansprüche

1. System (1) zur Beatmung von Patienten, umfassend eine Maske (2),
eine an der Maske(2) ausgebildete Anschlusseinrichtung (5) zur Verbindung der Maske (2) mit einem Luftschlauch,
eine Stirnstütze (7) mit einer Auflagefläche (17) zum Auflegen auf eine Stirn eines Patienten,
ein Bandsystem (10) zur Befestigung der Maske (2) an einem Kopf eines Patienten,
Befestigungsmittel (14) für das Bandsystem (10),
wobei die Stirnstütze (7) und die Befestigungsmittel (14) lösbar an der Maske (2) befestigbar sind, so dass das System (1) ohne Stirnstütze (7) und ohne Befestigungsmittel (14) als Anästhesiemaske (4) nutzbar ist und das System (1) mit Stirnstütze (7) und mit Befestigungsmittel (14) als Beatmungsmaske (3) nutzbar ist,
wobei die Position der Auflagefläche (17) der Stirnstütze (7) relativ zu der Maske (2) fixierbar einstellbar ist **dadurch gekennzeichnet,**
**dass** die Stirnstütze (7) eine im Volumen veränderliche, mit Luft gefüllte Auflageeinrichtung (18) aufweist, so dass mittels eines veränderbaren Volumens an Luft in der Auflageeinrichtung (18) die Position der Auflagefläche (17) der Stirnstütze (7) relativ zu der Maske (2) einstellbar ist, und
**dass** die Auflageeinrichtung (18) ein elastischer Ballon (19) ist und die Auflageeinrichtung (18) ein Ventil (20) zum Befüllen und Entleeren des Ballons (19) umfasst.

2. System nach Anspruch 1, **dadurch gekennzeichnet, dass** die Stirnstütze (7) und die Befestigungsmittel (14), insbesondere sämtliche Befestigungsmittel (14) als ein separates Bauteil (8) ausgebildet sind und/oder sämtliche Befestigungsmittel (14) lösbar an der Maske (2) befestigbar sind.

3. System nach Anspruch 2, **dadurch gekennzeichnet, dass** das Bauteil (8) an der Maske (2) lösbar befestigbar ist.

4. System nach Anspruch 2 oder 3, **dadurch gekennzeichnet, dass** das Bauteil (8) einen Ring (9) umfasst.

5. System nach einem oder mehreren der vorhergehenden Ansprüche, **dadurch gekennzeichnet, dass** die Maske (2) einen Stutzen (6), insbesondere als Anschlusseinrichtung (5) zur Verbindung der Maske (2) mit dem Luftschlauch, umfasst.

6. System nach Anspruch 5, **dadurch gekennzeichnet, dass** der Ring (9) des Bauteils (98) auf den Stutzen (6) aufschiebbar ist zur lösbaren Verbindung des Bauteils (8) mit der Maske (2).

7. System nach einem oder mehreren der vorhergehenden Ansprüche, **dadurch gekennzeichnet, dass** die Stirnstütze (7) und die Befestigungsmittel (14) mittels einer Schraub- oder Rastverbindung an der Maske (2) lösbar befestigbar sind.

8. System nach einem oder mehreren der vorhergehenden Ansprüche, **dadurch gekennzeichnet, dass** die Befestigungsmittel (14) als wenigstens ein Haken (15) und/oder wenigstens eine Öse (16) und/oder wenigstens ein Schlitz ausgebildet sind.

9. System nach einem oder mehreren der vorhergehenden Ansprüche, **dadurch gekennzeichnet, dass** das Bandsystem (10) ein Stirnband (12) und Seitenbänder (11) umfasst.

10. System nach einem oder mehreren der vorhergehenden Ansprüche, **dadurch gekennzeichnet, dass** das Bandsystem (10) elastisch ist.

11. System nach einem oder mehreren der vorhergehenden Ansprüche, **dadurch gekennzeichnet, dass** die Maske (2) und/oder die Stirnstütze (7) und/oder das Bandsystem (10) wenigstens teilweise, insbesondere vollständig, aus Kunststoff ausgebildet sind.

12. System nach einem oder mehreren der vorhergehenden Ansprüche, **dadurch gekennzeichnet, dass** die Befestigungsmittel (14) ausschließlich an dem separaten Bauteil (8) ausgebildet sind.

## Claims

1. A system (1) for respirating patients, comprising a mask (2) ;
a connection means (5) formed on the mask (2) for connecting the mask (2) with a flexible air tube;
a forehead support (7) with a support surface (17) for being placed on a forehead of a patient;
a strap system (10) for fastening the mask (2) to a head of a patient;
a fastening means (14) for the strap system,
wherein the forehead support (7) and the fastening means (14) can be detachably fastened to the mask (2), so that the system (1) without the forehead support (7) and without the fastening means (14) can be used as an anesthesia mask and that the system (1) with the forehead support (7) and the fastening means (14) can be used as a breathing mask (3);
the position of the support surface (17) of the forehead support (7) relative to the mask (2) is adjustable in a fixable manner,
**characterized in that**
the forehead support (7) has an air-filled support means (18) of variable volume, so that the position of the support surface (17) of the forehead support (7) relative to the mask (2) is adjustable by means of a variable volume of air in the support means (18); and
the support means (18) is an elastic balloon and that the support means (18) comprises a valve (20) for filling and emptying the balloon (19).

2. A system in accordance with claim 1, **characterized in that** the forehead support (7) and the fastening means (14), in particular all fastening means (14), are each formed as a separate component (8) and/or all fastening means (14) can be detachably fastened to the mask (2).

3. A system in accordance with claim 2, **characterized in that** the component (8) can be detachably fastened to the mask (2).

4. A system in accordance with claim 2 or 3, **characterized in that** the component (8) comprises a ring (9).

5. A system in accordance with one or several of the preceding claims, **characterized in that** the mask (2) comprises a connection piece (6), in particular as a connection means (5) for connecting the mask (2) to the flexible air tube.

6. A system in accordance with claim 5, **characterized in that** the ring (9) of the component (8) can be pushed onto the connection piece (6) for detachably connecting the component (8) to the mask (2).

7. A system in accordance with one or several of the preceding claims, **characterized in that** the forehead support (7) and the fastening means (14) are detachably fastened to the mask (2) by means of a screw or locking connection.

8. A system in accordance with one or several of the preceding claims, **characterized in that** the fastening means (14) are formed as at least one hook (15) and/or at least one eyelet (16) and/or as at least one slot.

9. A system in accordance with one or several of the preceding claims, **characterized in that** the strap system comprises a head strip (12) and side straps (11).

10. A system in accordance with one or several of the preceding claims, **characterized in that** the strap system (10) is elastic.

11. A system in accordance with one or several of the preceding claims, **characterized in that** the mask (2) and/or forehead support (7) and/or the strap system are made at least partly, in particular completely, of plastic.

12. A system in accordance with one or several of the preceding claims, **characterized in that** the fastening means (14) is formed exclusively on the separate component (8).

## Revendications

1. Système (1) de ventilation artificielle de patients, comprenant un masque (2),
un dispositif de raccordement (5) réalisé sur le masque (2) et destiné à relier le masque (2) à un tuyau d'air,
un élément d'appui frontal (7) doté d'une surface d'appui (17) destinée à être posée sur le front d'un patient,
un système de bandes (10) destiné à fixer le masque (2) sur la tête d'un patient, des moyens de fixation (14) pour le système de bandes (10),
l'élément d'appui frontal (7) et les moyens de fixation (14) pouvant être fixés de manière amovible au masque (2), de sorte que le système (1) peut être utilisé sans élément d'appui frontal (7) et sans moyens de fixation (14), en tant que masque d'anesthésie (4), et le système peut être utilisé avec l'élément d'appui frontal (7) et les moyens de fixation (14), en tant que masque de ventilation (3),
la position de la surface d'appui (17) de l'élément d'appui frontal (7) par rapport au masque (2) pouvant être réglée de manière fixe,
**caractérisé en ce que**
l'élément d'appui frontal (7) présente un dispositif d'appui (18) rempli d'air, à volume variable, de sorte que la position de la surface d'appui (17) de l'élément d'appui frontal (7) par rapport au masque (2) peut être réglée au moyen d'un volume d'air variable dans le dispositif d'appui (18), et
**en ce que** le dispositif d'appui (18) est un ballon (19) élastique, et le dispositif d'appui (18) comprend une valve (20) pour remplir et vider le ballon (19).

2. Système selon la revendication 1, **caractérisé en ce que** l'élément d'appui frontal (7) et les moyens de fixation (14), en particulier tous les moyens de fixation (14), sont réalisés sous forme de composant (8) séparé, et/ou tous les moyens de fixation (14) peuvent être fixés de manière amovible au masque (2).

3. Système selon la revendication 2, **caractérisé en ce que** le composant (8) peut être fixé de manière amovible au masque (2).

4. Système selon la revendication 2 ou 3, **caractérisé en ce que** le composant (8) comprend une bague (9).

5. Système selon une ou plusieurs des revendications précédentes, **caractérisé en ce que** le masque (2) comprend une tubulure (6), en particulier en tant que dispositif de raccordement (5), pour relier le masque (2) au tuyau d'air.

6. Système selon la revendication 5, **caractérisé en ce que** la bague (9) du composant (8) peut être glissée sur la tubulure (6), afin de relier le composant (8) de manière amovible au masque (2).

7. Système selon une ou plusieurs des revendications précédentes, **caractérisé en ce que** l'élément d'appui frontal (7) et les moyens de fixation (14) peuvent être fixés de manière amovible au masque (2), à l'aide d'un assemblage à vis ou à encliquetage.

8. Système selon une ou plusieurs des revendications précédentes, **caractérisé en ce que** les moyens de fixation (14) sont réalisés sous la forme d'au moins un crochet (15) et/ou d'au moins un oeillet (16) et/ou d'au moins une fente.

9. Système selon une ou plusieurs des revendications précédentes, **caractérisé en ce que** le système de bandes (10) comprend une bande frontale (12) et des bandes latérales (11).

10. Système selon une ou plusieurs des revendications précédentes, **caractérisé en ce que** le système de bandes (10) est élastique.

11. Système selon une ou plusieurs des revendications précédentes, **caractérisé en ce que** le masque (2) et/ou l'élément d'appui frontal (7) et/ou le système de bandes (10) est/sont réalisé(s) au moins en partie, notamment en totalité, en matière plastique.

12. Système selon une ou plusieurs des revendications précédentes, **caractérisé en ce que** les moyens de fixation (14) sont réalisés exclusivement sur le composant (8) séparé.
